Europäisches Patentamt

European Patent Office

Office européen des brevets

(11) Numéro de publication: **0 356 291 B1**

(12) **FASCICULE DE BREVET EUROPEEN**

(45) Date de publication de fascicule du brevet: **22.06.94**

(51) Int. Cl.5: **C12P 7/42**, C12P 17/04, C07D 315/00

(21) Numéro de dépôt: **89402205.2**

(22) Date de dépôt: **03.08.89**

Le dossier contient des informations techniques présentées postérieurement au dépôt de la demande et ne figurant pas dans le présent fascicule.

(54) **Procédé de production microbiologique de décanolide gamma (R) et d'octanolide gamma (R).**

(30) Priorité: **04.08.88 IT 6774288**

(43) Date de publication de la demande:
**28.02.90 Bulletin 90/09**

(45) Mention de la délivrance du brevet:
**22.06.94 Bulletin 94/25**

(84) Etats contractants désignés:
**AT BE CH DE ES FR GB GR IT LI LU NL SE**

(56) Documents cités:
**EP-A- 0 258 993**
**WO-A-83/01072**

**PATENT ABSTRACTS OF JAPAN, vol. 9, no. 241 (C-306)[1964], 27 septembre 1985&NUM;**

**PATENT ABSTRACTS OF JAPAN, vol. 9, no. 196 (C-297)[1919], 13 août1985&NUM;**

(73) Titulaire: **PERNOD-RICARD**
**142, Boulevard Haussmann**
**F-75008 Paris(FR)**

(72) Inventeur: **Cardillo, Rosanna**
**Via Pietro da Cortona 7**
**I-Milano(IT)**
Inventeur: **Fuganti, Claudio**
**Via G.B. Nazari 8**
**I-Milano(IT)**
Inventeur: **Sacerdote, Guiseppe**
**Via S. Pio V. 84**
**I-Torino(IT)**
Inventeur: **Barbeni, Massimo**
**Via Baretti 19**
**I-Torino(IT)**
Inventeur: **Cabella, Paolo**
**Corso Sommellier 17**
**I-Torino(IT)**
Inventeur: **Squarcia, Francesco**
**Via Tagliacozzi 9**
**I-Bologna(IT)**

(74) Mandataire: **Warcoin, Jacques et al**
**Cabinet Régimbeau**
**26, avenue Kléber**
**F-75116 Paris (FR)**

## Description

La présente invention a trait à un procédé de production par des moyens microbiologiques d'une gamma-lactone optiqument active, en particulier de la lactone de l'acide hydroxy-4 décanoïque (4R) (gamma-décanolide ou gamma-décalactone) et de la lactone de l'acide hydroxy-4 octanoïque (4R), (gamma-octanolide).

Les lactones citées ci-dessus sont des substances volatiles qui sont très importantes en termes pratiques, car elles sont présentes dans les aliments naturels dont elles constituent une partie de l'arôme.

Il n'est pas économique d'extraire ces composés de sources naturelles parce qu'ils sont en général présents en petites quantités et parce qu'il est difficile des les séparer physiquement des autres composés volatils qui s'y trouvent en même temps.

Le brevet américain US-A-4.560.656 décrit un procédé de production de gamma-décalactone qui exploite la capacité d'espèces des microorganismes des genres Candida, Aspergillus, Geotrichum et Yarrowia de dégrader de façon oxydative l'acide ricinoléique que l'on ajoute à la culture sous cette forme ou sous la forme d'huile de ricin, en présence ou en l'absence d'une lipase externe, pour donner l'acide hydroxy-4 décanoïque (4R) ou la lactone correspondante directement.

Le fait que l'acide gamma-hydroxydécanoïque est un intermédiaire dans la dégradation oxydative de l'acide ricinoléique par des souches appartenant au genre Candida a déjà été étudié par Okui et coll. (J. Biochemistry, 54, 536-540, 1963).

Le brevet américain US-A-4,396,715 décrit un procédé d'obtention d'un arôme comprenant des octalactones, des nonalactones eu des décalactones par incubation d'un microorganisme du genre Pityrosporum.

Les brevets japonais JP-A-60100508 et JP-A-6066PP1 décrivent des microorganismes Pichia Farinosa utilisés pour modifier de l'huile de ricin pour l'obtention d'une γ-décalactone.

La présente invention a pour object un procédé de production de gamma-décanolide et/ou de gamma-octanolide optiquement active qui comprend la mise en contact d'une huile végétale, de l'un de ses hydrolysats et/ou d'acide ricinoléique naturel avec une culture en phase de croissance d'un microorganisme sélectionné parmi le groupe comprenant Aspergillus niger (CBS 102.12), Cladosporium suaveolens (CBS 157.58), Pichia etchellsii - (CBS 2011) et Phanerochaete chrysosporium (CBS 57863).

Des modes de réalisations préférés sont contenus dans les revendications 4, 5 et 6.

On préfère utiliser en particulier de l'huile de ricin, de l'huile de tournesol, leurs hydrolysats ou de l'acide ricinoléique pour obtenir du gamma-décanolide.

On préfère utiliser de l'huile de noix de coco en contact avec une culture en phase de croissance de Cladosporium suaveolens pour obtenir du gamma-octanolide.

L'emploi de l'huile de tournesol constitue un avantage économique considérable dans le procédé selon l'invention parce qu'elle est peu coûteuse. Un avantage supplémentaire réside en la possibilité d'utiliser de l'acide ricinoléique naturel qui est suffisamment non toxique envers les microorganismes cités ci-dessus pour ne pas inhiber leur métabolisme. L'emploi d'acide ricinoléique présente des avantages particuliers dans la phase séparation de la lactone désirée, parce qu'il est bien connu qu'elle est particulièrement simple en milieu acide.

La lactone désirée est obtenue à partir des matériaux de départ mentionnés ci-dessus dans des conditions de culture variables et en un temps relativement court, compris entre 24 et 60 heures. De façon typique, on maintient les microorganismes en contact avec les matériaux de départ mentionnés ci-dessus à des températures comprises entre 20 et 30°C inclus.

Le milieu nutritif dans lequel on cultive les microorganismes est du type classique, et, à ce sujet, il faudra se référer aux exemples qui suivent. La culture se déroule sous agitation en ce qui concerne la phase de dégradation au cours de laquelle on obtient la lactone tandis que la production de la biomasse utilisée en tant que préinoculum peut se dérouler avec ou sans agitation, et peut être contrôlée en ce qui concerne la production du composé désiré, par des méthodes techniques standard telles que la CPV (chromatographie en phase vapeur), CCM (chromatographie en couche mince), HPLC (chromatographie liquide sous haute pression), IR (infrarouge) et RMN (résonance magnétique nucléaire). Lorsque l'analyse montre que la production a atteint un maximum, on extrait la lactone désirée. Comme on le sait (voir l'article de I.L. Finar dans Organic Chemistry Vol. I, pages 427-428 par exemple) la forme lactone du composé désiré est sujette à un échange avec l'acide gamma-hydroxy correspondant, et l'extraction de la lactone implique donc la transformation de l'acide gamma-hydroxy en la lactone correspondante.

1) Pour extraire la lactone, on filtre le milieu de culture sur de la Célite (R), on lave à l'acetate d'éthyle et on extrait la phase aqueuse, de pH acide, de préférence de pH 5, de préférence deux fois à l'aide d'acétate d'éthyle. On extrait les phases organiques combinées deux fois à l'aide d'une solution de carbonate de potassium à 5 pour cent pour éliminer les portions acides.

On évapore ensuite la phase organique, séchée sur sulfate de sodium, et on distille le résidu à 150°C et sous 1-3 mm de Hg (133,3 à 399,9 Pa) pour obtenir le gamma-décanolide ou le gamma-octanolide.

2) Pour améliorer le rendement en lactone, on peut lactoniser l'acide $\gamma$-hydroxydécanoïque dans le milieu en amenant le pH entre 1 et 5, de préférence entre 1 et 3, par addition d'un acide approprié et en chauffant le milieu acidifié à une température comprise entre 50°C et 110°C, de préférence comprise entre 90 et 100°C, pendant une période comprise entre environ 10 minutes et 2 heures selon la température. On peut séparer la lactone du milieu par entraînement à la vapeur d'eau à partir du milieu acidifié.

Dans les exemples qui suivent, la quantité de lactone obtenue est exprimée en pourcentage tel qu'il a été obtenu par analyse par chromatographie en phase vapeur (CPV).

## Exemple 1

On inocule une suspension d'"Aspergillus niger" (CBS 102.12) dans un flacon Erlenmeyer de 300 ml contenant une solution de nutrient de Merck à 2%, de Tween® 80 à 0,02% et de 5 g d'acide ricinoléique de pH 7, qui a été stérilisée dans un autoclave pendant 10 minutes à 120°C; ceci est maintenu à 27-30°C pendant une période comprise entre deux et cinq jours et on agite tout le temps. On prélève des échantillons pendant cette période, et on détermine par chromatographie en phase vapeur (CPV) le gamma-décanolide dans l'extrait organique qui est obtenu en agitant l'échantillon avec de l'éther éthylique. On trouve une teneur en gamma-décanolide comprise entre 4 et 12%.

## Exemple 2

On répète la procédure de l'exemple 1 mais avec du Cladosporium suaveolens (CBS 157.58). On trouve une teneur en gamma-décanolide comprise entre 4 et 10%.

## Exemples 3 et 4

On répète les procédures des exemples 1 et 2 mais on utilise 6 g d'huile de ricin au lieu d'acide ricinoléique. On obtient approximativement 5% de gamma-décanolide dans les deux cas après quatre jours d'incubation.

## Exemple 5: procédé de séparation

On suit les procédures décrites dans les exemples 1 et 2 avec 5 flacons Erlenmeyer, en utilisant en tout 25 g d'acide ricinoléique; après 48 heures, on filtre les cultures sur de la Célite et on lave la Célite à l'aide d'acétate d'éthyle.

On extrait deux fois la phase aqueuse de pH 5 à l'aide d'acétate d'éthyle. Les phases organiques d'extraction et de lavage de la Célite (220 ml) sont extraites deux fois à l'aide de 150 ml d'une solution de $K_2CO_3$ à 5%. On sèche la phase organique sur du sulfate de sodium et on évapore à sec. Une chromatographie en couche mince (CCM) indique la présence de gamma-décanolide et de nombreux autres produits mobiles (éluant: 7 parties d'hexane, 3 parties, d'acétate d'éthyle). Cette substance est distillée dans un ballon à fond rond pour donner environ 350 mg de gamma décanolide, 99.5% en chromatographie en phase vapeur (CPV) où $[\alpha]_D^{20}$ = +49 (c l, méthanol).

## Exemple 6

On inocule le contenu d'une éprouvette de Cladosporium suaveolens, cultivé sur MPGA (20 g/l d'extrait de malt, 5 g/l de peptone, 20 g/l de glucose, 15 g/l de gélose) à 24°C pendant sept jours, dans un flacon Erlenmeyer de 300 ml contenant 50 ml de MPGB (20 g/l d'extrait de malt, 5 g/l de peptone, 20 g/l de glucose, et complété avec de l'eau). On place le flacon dans une enceinte contrôlée thermostatiquement à 30°C et on maintient sous agitation pendant deux jours .

On se sert de ce préinoculum pour ensemencer des flacons de 300 ml contenant 50 ml de MPGB, en mettant 5 ml dans chaque flacon.

On cultive ces flacons pendant quatre jours à 30°C sans agitation, point auquel on remplace le milieu par 100 ml d'extrait de viande (20 g/l), 5 g d'acide ricinoléique et 0,2% de Tween® et on agite à 30°C.

On surveille la production de gamma-décanolide au moyen d'extractions successives à la suite de deux, trois, quatre, cinq et sept jours.

Extraction à la suite de quatre jours: poids de l'extrait brut: 3 g, analyse par chromatographie en phase vapeur (CPV): 20% de gamma-décanolide, gamma-décanolide isolé par distillation: 0.5 g.

Extraction à la suite de cinq jours: poids de l'extrait brut: 3 g, chromatographie en phase vapeur (CPV): 17%

Extraction à la suite de sept jours: poids de l'extrait brut: 3 g, chromatographie en phase vapeur (CPV): 17%.

Exemple 7

On inocule du Cladosporium suaveolens prélevé d'une éprouvette de MPGA cultivé pendant deux jours, dans un flacon Erlenmeyer de 300 ml contenant 100 ml de milieu préparé à l'aide d'extrait de viande (5 g/l) et de 0,2% de Tween®. On le laisse à cultiver pendant deux jours à 30°C sous agitation.

A ce point, on ajoute 1 ml d'acide ricinoléique, ce qui amène le pH à 6,5. On agite le mélange à 30°C pendant 18 jours. On obtient 1,1 g d'extrait brut, qui se révèle comme contenant 40% de gamma-décanolide, à partir de quatre flacons (ce qui équivaut à 5 g d'acide ricinoléique).

Exemple 8

On ajoute un inoculum de Cladosporium suaveolens prélevé d'une éprouvette dans un flacon Erlenmeyer de 300 ml contenant un milieu comprenant de l'extrait de viande (5 g/l) et 0,2% de Tween® et 1 g d'acide ricinoléique (stérilisés tous ensemble). A la suite de neuf jours sous agitation à 30°C, le pH est devenu égal à 6,5. On extrait le contenu des deux flacons (correspondant à 2 g d'acide ricinoléique) et l'on obtient 0,33 g d'extrait brut contenant 62% de gamma-décanolide.

Exemple 9

On répète la procédure de l'exemple 6 jusqu'au point auquel on remplace le milieu à l'aide de 100 ml d'extrait de viande. A ce point, on ajoute 5 g d'huile de tournesol et 0,2% de Tween au lieu de l'acide ricinoléique. On agite le mélange et puis on l'extrait à la suite de 15 jours: extrait brut: 2,6 g; % de lactone: 31% en chromatographie en phase vapeur (CPV) (38% après une lactonisation). On distille cette substance à 150°C et sous 2 mm Hg pour obtenir 0,350 g de gamma-décanolide pur.

Exemple 10

On ensemence un flacon Erlenmeyer de 300 ml contenant 100 ml de bouillon nutritif (20 g/l), 0,2 g de Tween® et 5 g d'acide ricinoléique à l'aide d'une préinoculum de Pichia etchellsii cultivé sur du GYP (50 g/l de glucose, 10 g/l d'extrait de levure, 10 g/l de peptone) pendant 24 heures. Ce préinoculum dans le flacon avait été inoculé à partir d'une éprouvette sur du MPGA. On avait laissé cultiver le contenu de l'éprouvette pendant deux jours à 24°C. Le pH est de 6.

On cultive le mélange à 30°C sous agitation; à la suite de six jours on extrait deux flacons (correspondant à 10 g d'acide ricinoléique). L'extrait brut pèse 7 g et contient 3,8% de gamma-décanolide.

A la suite de sept jours, on extrait deux flacons, correspondant à 10 g d'acide ricinoléique, pour obtenir un extrait brut que l'on distille dans un ballon à fond rond. On obtient 0,18 g de gamma-décanolide.

A la suite de neuf jours, on extrait quatre flacons, correspondant à 20 g d'acide ricinoléique. L'extrait brut pèse 4,4 g. On distille 2,2 g de cette substance dans un ballon à fond rond pour obtenir 0,1 g de gamma-décanolide (43%) tandis qu'avant la distillation, l'extrait brut contenait 4,4% de gamma-décanolide.

On extrait quatre flacons à la suite de 15 jours. L'extrait brut est repris par de l'hexane et extrait ensuite à l'aide de méthanol/$K_2CO_3$ à 5% 1:1. La phase hexanique est concentrée pour obtenir environ un gramme de substance contenant 12% de lactone.

Exemple 11

On inocule un flacon Erlenmeyer de 300 ml contenant 100 ml de milieu à base d'extrait de viande (20 g/l), 0,2 g de Tween® et 2 g d'acide ricinoléique (stérilisés tous ensemble) à l'aide de Pichia etchellsii $5.10^8$ cellules/ml provenant d'une préculture (exemple 10).

A la suite de cinq jours sous agitation à 30°C, on acidifie un flacon correspondant à 2 g d'acide ricinoléique à pH 3 et on le chauffe à 100°C. On continue la distillation pendant 2 heures et on extrait le condensat aqueux à l'aide d'hexane pour donner 0,2 g de $\gamma$-décanolide (R).

Exemple 12

On inocule un flacon Erlenmeyer de 300 ml contenant 100 ml de milieu comprenant de l'extrait de viande (5 g/l), 0,2% de Tween® et 1 g d'acide ricinoléique (stérilisés tous ensemble) à l'aide de Pichia etchellsii directement à partir d'une éprouvette.

On extrait deux flacons correspondant à 2 g d'acide ricinoléique à la suite de deux jours. L'extrait brut pèse 0,32 g et contient 23% de gamma-décalactone.

On extrait deux flacons supplémentaires à la suite de cinq jours. Extrait brut 0,36 g, 5,3% de gamma-décanolide.

Exemple 13

On inocule un flacon Erlenmeyer de 300 ml contenant 100 ml de milieu comprenant de l'extrait de viande (5 g/l), 0,2% de Tween® et 1 g d'huile de noix de coco (dont on a prouvé qu'elle était dépourvue de gamma-octanolide) à l'aide de Cladosporium suaveolens directement à partir d'une éprouvette. On effectue l'extraction à la suite de cinq jours et on obtient environ 100 mg de gamma-octanolide.

## Revendications

1. Procédé de production d'une gamma-lactone optiquement active sélectionnée parmi la gamma-décalactone et la gamma-octalactone qui comprend la mise en contact d'une huile végétale, de l'un de ses hydrolysats ou d'acide ricinoléique avec une culture en phase de croissance d'un microorganisme sélectionné parmi le groupe comprenant Aspergillus niger, Cladosporium suaveolens, Phanerochaete chrysosporium et Pichia etchellsii.

2. Procédé selon la revendication 1, dans lequel l'huile végétale est choisie parmi l'huile de ricin, l'huile de tournesol et l'huile de noix de coco.

3. Procédé selon la revendication 1, dans lequel on se sert de Cladosporium suaveolens et d'huile de noix de coco pour obtenir du gamma-octanolide.

4. Procédé selon la revendication 1 et la revendication 2 dans lequel l'huile végétale est hydrolysée par voie enzymatique à la lipase.

5. Procédé selon la revendication 1 dans lequel l'acide γ-hydroxydécanoïque est lactonisé par l'action de la chaleur à pH acide.

6. Procédé selon la revendication 1 et 5 dans lequel la γ-décalactone (R) est séparée par entraînement à la vapeur d'eau à partir du milieu acidifié.

## Claims

1. A process for the production of an optically active gamma-lactone selected from gamma-decalactone and gamma-octalactone which comprises contacting a vegetable oil, one of its hydrolyzates or ricinoleic acid with a culture in the growth phase of a microorganism selected from the group consisting of Aspergillus niger, Cladosporium suaveolens, Phanerochaete chrysosporium and Pichia etchellsii.

2. A process as claimed in claim 1, in which the vegetable oil is selected from castor oil, sunflower oil and coconut oil.

3. A process as claimed in claim 1, characterized in that Cladosporium suaveolens and coconut oil are used to obtain gamma-octanolide.

4. A process as claimed in claims 1 and 2, in which the vegetable oil is enzymatically hydrolyzed with lipase.

5. A process as claimed in claim 1, in which gamma-hydroxydecanoic acid is lactonized by exposure to heat at an acidic pH.

6. A process as claimed in claims 1 and 5, in which the gamma-decalactone (R) is separated from the acidified medium by stripping with steam.

## Patentansprüche

1. Verfahren zur Herstellung eines optisch aktiven γ-Lactons, ausgewählt unter γ-Decalacton und γ-Octalacton, umfassend das In-Kontakt-Bringen eines Pflanzenöles, eines seiner Hydrolysate oder von Ricinoleinsäure mit einer Wachstumskultur eines Mikroorganismus, ausgewählt aus der Aspergillus niger, Cladosporium suaveolens, Phanerochaete chrysosporium und Pichia etchellsii umfassenden Gruppe.

2. Verfahren nach Anspruch 1, bei dem das Pflanzenöl unter Ricinusöl, Sonnenblumenöl und Kokosnußöl ausgewählt wird.

3. Verfahren nach Anspruch 1, bei dem man Cladosporium suaveolens und Kokosnußöl einsetzt, um γ-Octanolid zu erhalten.

4. Verfahren nach Anspruch 1 und Anspruch 2, bei dem das Pflanzenöl auf enzymatischem Wege mit Lipase hydrolysiert wird.

5. Verfahren nach Anspruch 1, bei dem die γ-Hydroxydecansäure durch Einwirkung von Wärme im sauren pH-Bereich lactonisiert wird.

6. Verfahren nach Anspruch 1 und 5, bei dem das γ-Decalacton (R) mit Hilfe von Wasserdampf aus dem angesäuerten Medium abgetrennt wird.